**Europäisches Patentamt**

**European Patent Office**

**Office européen des brevets**

(19)

(11) Numéro de publication : **0 193 480**
**B1**

(12) **FASCICULE DE BREVET EUROPÉEN**

(45) Date de publication du fascicule du brevet :
17.08.88

(21) Numéro de dépôt : 86430001.7

(22) Date de dépôt : 28.01.86

(51) Int. Cl.⁴ : **A 61 N   1/30**

(54) **Procédé et dispositif pour traitements amincissants par ionophorèse ou par galvanothérapie.**

(30) Priorité : 29.01.85 FR 8501337

(43) Date de publication de la demande :
03.09.86 Bulletin 86/36

(45) Mention de la délivrance du brevet :
17.08.88 Bulletin 88/33

(84) Etats contractants désignés :
AT BE CH DE GB IT LI LU NL SE

(56) Documents cités :
DE-A- 2 021 295
FR-A- 1 541 165
US-A- 3 610 250

(73) Titulaire : **Barret, Jean Pierre**
**29, boulevard P. et M. Curie**
**F-13220 Chateauneuf-les Martigues (FR)**

(72) Inventeur : **Barret, Jean Pierre**
**29, boulevard P. et M. Curie**
**F-13220 Chateauneuf-les Martigues (FR)**

(74) Mandataire : **Marek, Pierre**
**28 & 32 rue de la Loge**
**F-13002 Marseille (FR)**

## Description

La présente invention concerne un procédé de traitement amincissant par ionophorèse, c'est-à-dire un procédé de traitement amincissant appliqué dans les instituts de beauté et/ou de soins esthétiques et utilisant le passage de courants électriques à travers le corps. Elle vise aussi un dispositif pour l'application de traitements amincissants par ionophorèse ou de traitements amaigrissants par galvanothérapie.

On connaît des dispositifs de traitement amincissant ou amaigrissant par ionophorèse ou par électrothérapie ou galvanothérapie, permettant de faire passer un courant électrique continu à travers certaines zones du corps humain et comprenant principalement un générateur de courant continu, éventuellement doublé d'un générateur de courant pulsé, et deux ou plus de deux électrodes destinées à être appliquées sur la peau et constituées par des blocs de caoutchouc conducteur.

La mise en œuvre des méthodes actuelles de traitements utilisant de tels dispositifs a notamment pour inconvénients : une efficacité assez médiocre, une durée de traitement relativement longue, et une fréquente irritation de la peau aux emplacements d'application des électrodes.

Ces inconvénients découlent plus particulièrement :

— du fait que les dispositifs de traitement par ionophorèse ou par galvanothérapie connus ne permettent qu'une application très localisée non adaptée à la forme des différentes parties du corps ;

— du fait que ces dispositifs entraînent la création, entre leurs deux électrodes, d'un passage préférentiel du courant électrique dans le corps.

Cette concentration ou focalisation du courant électrique a évidemment pour conséquences de limiter les effets du traitement aux zones de passage du courant et d'entraîner fréquemment une inflammation de la peau aux emplacements de contact des électrodes.

Le procédé et le dispositif qui font l'objet de la présente invention ont plus particulièrement pour but de remédier aux inconvénients ci-dessus.

Selon l'invention, cet objectif est atteint grâce à un procédé suivant lequel le courant électrique est envoyé à travers la ou les parties du corps à traiter, au moyen d'électrodes souples et enveloppantes exécutées en caoutchouc conducteur et disposées autour de la ou desdites parties, ces électrodes enveloppantes étant conformées pour épouser le plus étroitement possible les parties du corps autour desquelles elles sont placées.

Le dispositif selon l'invention comporte des électrodes exécutées en caoutchouc conducteur de l'électricité, ces électrodes étant principalement remarquables par le fait qu'elles sont constituées par des enveloppes souples aptes à entourer des parties déterminées du corps humain et convenablement conformées pour épouser le plus étroitement possible les parties du corps autour desquelles elles sont destinées à être placées.

Grâce au procédé et au dispositif selon l'invention, on obtient des traitements amincissants ou amaigrissants plus efficaces dont la durée est abrégée, l'application de ces traitements ne provoquant aucune irritation de la peau. En effet, le bon contact existant entre les électrodes enveloppantes et les parties du corps traitées, l'importance des surfaces de la peau en contact avec ces électrodes et l'enveloppement complet des parties du corps sur lesquelles elles sont placées, font qu'il n'y a aucun cheminement ou passage préférentiel du courant électrique à travers lesdites parties, ni concentration ou focalisation du courant au niveau des zones de contact et de passage du courant. D'autre part, les électrodes enveloppantes du dispositif selon l'invention permettent d'obtenir un meilleur équilibre des potentiels.

Les buts, caractéristiques et avantages susmentionnés, et d'autres encore, ressortiront mieux de la description qui suit et des dessins annexés dans lesquels :

La figure 1 est une vue de face du dispositif selon l'invention réalisé sous forme d'une combinaison morcelable dont chaque élément constitue l'une des électrodes enveloppantes de ce dispositif.

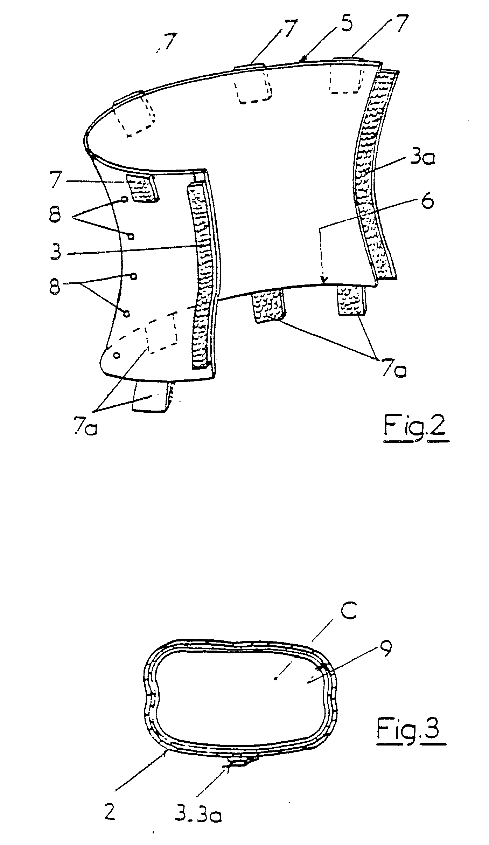

La figure 2 est une vue en perspective d'une électrode enveloppante selon l'invention qui affecte, dans ce cas, la forme d'une ceinture abdominale.

La figure 3 est une vue en coupe transversale montrant le positionnement et la fixation d'une électrode enveloppante réalisée sous forme d'une ceinture abdominale placée autour de l'abdomen d'une utilisatrice.

La figure 4 est une vue analogue à la figure 1 et montrant l'application de quelques éléments d'une combinaison similaire, sur certaines parties du corps seulement.

La figure 5 est une vue du schéma électrique d'un dispositif utilisant les électrodes enveloppantes représentées à la figure 4.

On se réfère aux dits dessins pour décrire un exemple avantageux, quoique nullement limitatif, de réalisation du dispositif et de mise en œuvre du procédé selon l'invention.

Ce dispositif comprend un générateur de courant continu 1, par exemple de 0 à 80 volts, et au moins deux électrodes. Ces électrodes 2 sont exécutées à l'aide d'une feuille de caoutchouc conducteur ou autre élastomère conducteur de l'élctricité et constituées, par exemple, par une poudre de carbone ou de graphite liée par du caoutchouc naturel pur.

Selon l'invention, ces électrodes sont en premier lieu remarquables par le fait qu'elles affectent la forme d'enveloppes souples aptes à entourer complètement des parties déterminées du corps humain et convenablement conformées pour épouser le plus étroitement possible les parties du corps C autour desquelles elles sont destinées à être placées et fixées durant l'application du traitement.

Selon une autre caractéristique de l'invention, chaque électrode souple et enveloppante constitue l'un des composants ou parties d'un ensemble morcelable apte à recouvrir et à entourer complètement une ou des parties plus ou moins étendues du corps (tronc, bras, jambes, bassin et cuisse, etc.) et, de manière préférée, l'un des composants ou parties d'une combinaison morcelable apte à envelopper complètement le corps, du cou aux chevilles.

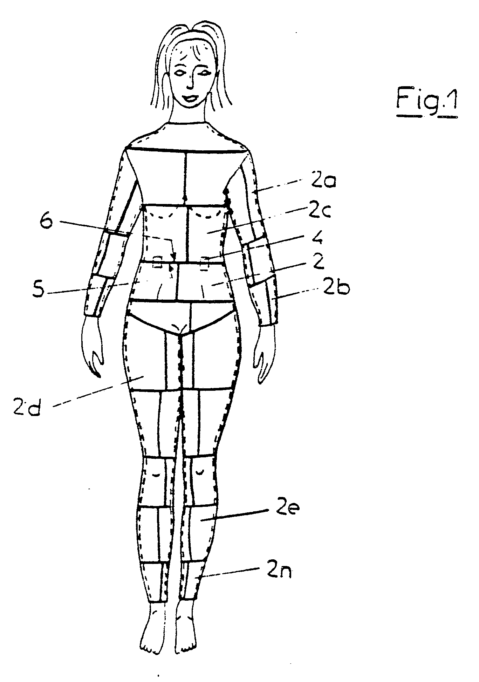

On a représenté, à la figure 1, un exemple de réalisation d'une telle combinaison morcelable qui est composée d'une pluralité d'électrodes enveloppantes juxtaposées 2, 2a, 2b, 2c, 2d, 2e,..., 2n, de sorte que lorsqu'elle est utilisée en totalité, elle enveloppe quasi complètement le corps traité dont elle épouse étroitement les formes.

Chaque électrode enveloppante est constituée par une feuille souple de caoutchouc ou autre élastomère conducteur, pleine ou ajourée, conformée, par moulage ou autrement, pour pouvoir reproduire, approximativement, après mise en place et fixation, la forme de la partie du corps autour de laquelle elle est appelée à être placée.

Au moins deux bords opposés de chaque électrode enveloppante 2, 2a, 2b, 2c, 2d, 2e,..., 2n, sont munis de moyens complémentaires d'assemblage rapide 3, 3a, tels que, par exemple, système à boucles et crochets connu sous la marque déposée « Velcro », boutons-pression, fermetures à glissières, etc.

D'autre part, le ou les bords 5, 6, de chaque électrode enveloppante destiné(s) à être placé(s) au contact ou à proximité du ou des bords d'une ou de deux électrodes contiguës, est ou sont également pourvu(s) de moyens d'assemblage rapide 7, 7a permettant de lier entre elles les différentes électrodes voisines de l'ensemble morcelable.

Chaque électrode enveloppante 2 peut être encore équipée d'une pluralité de bornes de connexion 8 permettant de la relier électriquement, au moyen d'un fil conducteur de raccordement équipé de fiches correspondantes, à une ou plusieurs autres électrodes enveloppantes de l'ensemble morcelable ou modulable. Cette opération peut également être obtenue par un raccordement dit « volant » au moyen de pinces crocodiles ou autres.

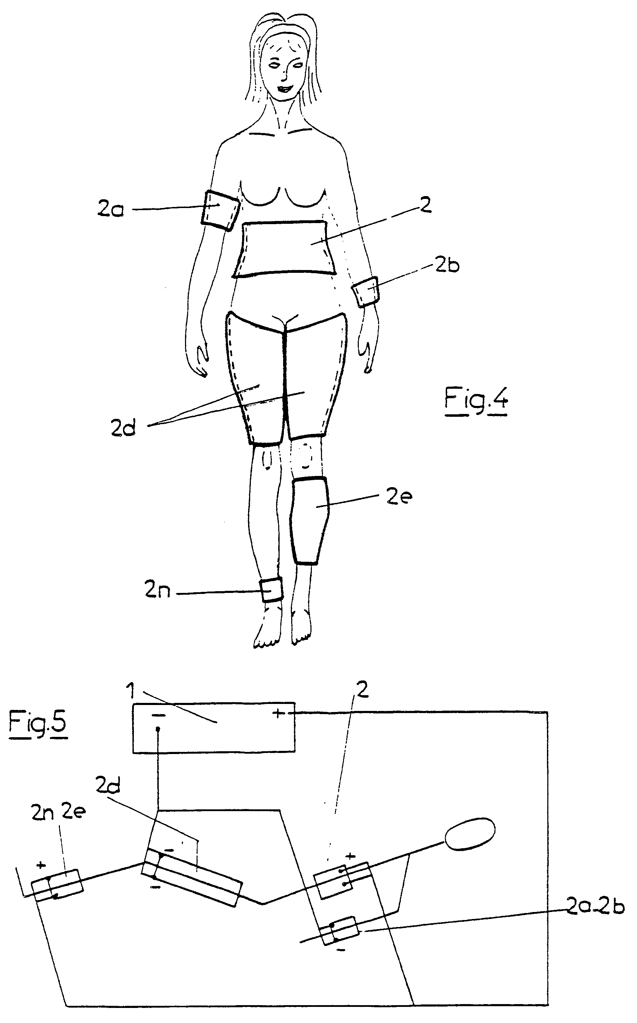

Il est possible d'utiliser, en fonction des besoins, la totalité des électrodes enveloppantes constituant l'ensemble morcelable, comme le montre la figure 1, ou un ou certains de ces éléments seulement, comme cela est représenté à la figure 4.

On précise que le corps étant assimilé à un conducteur, chaque fois que l'on passe d'une électrode enveloppante à une autre, d'un bout à l'autre du conducteur, les polarités de ces électrodes sont inverses.

Ainsi, comme le montre le schéma électrique de la figure 5 qui correspond aux électrodes enveloppantes 2, 2a, 2b, 2d, 2n, la borne + du générateur est raccordée à la ceinture abdominale 2, et aux enveloppes de cheville 2n et de mollet 2e, tandis que la borne dudit générateur est raccordée aux enveloppes de cuisses 2d, de bras 2a et de poignet 2b, les polarités étant inversées après une certaine durée de traitement, par exemple au bout de 15 m.

Les électrodes enveloppantes peuvent être utilisées en combinaison ou non avec des substances ou solutions ionisables amincissantes ou amaigrissantes, connues en soi, appliquées sur la peau avant mise en place desdites électrodes et envoi du courant électrique à travers le corps. Ces substances ou solutions destinées à pénétrer dans les couches cutanées et sous-cutanées du corps, améliorent l'efficacité du traitement et/ou en complètent les effets.

De manière avantageuse, une couche 9 de matière végétale est disposée autour de la ou des parties du corps C à traiter, entre la peau et la face interne de chaque électrode enveloppante, avant la mise en place de celle-ci.

De manière préférée, cette couche de matière végétale est constituée par un tissu de coton se présentant sous forme de bandelette du genre bande velpeau enroulée autour de la partie du corps à traiter.

Cette couche de coton ou autre matière végétale a notamment pour effets bénéfiques :

— d'éviter les réactions allergiques au caoutchouc qui se traduisent par des inflammations de la peau et des démangeaisons ;

— de mieux répartir le passage du courant entre les électrodes enveloppantes et le corps.

## Revendications

1. Procédé de traitement amincissant par ionophorèse, utilisant le passage de courants électriques à travers le corps, au moyen d'électrodes souples appliquées sur la peau, caractérisé en ce que le courant électrique est envoyé à travers le corps, au moyen d'électrodes enveloppantes (2, 2a, 2b, 2c, 2d, 2e,..., 2n) réalisées en caoutchouc conducteur et disposées autour de la ou des parties du corps à traiter, ces électrodes enveloppantes étant conformées pour épouser le plus étroitement possible la ou lesdites parties du corps autour desquelles elles sont placées.

2. Procédé de traitement amincissant par ionophorèse selon la revendication 1, caractérisé en ce qu'une substance ou solution ionisable amincissante destinée à compléter et/ou à améliorer l'efficacité du traitement, est appliquée sur la peau avant l'envoi du courant électrique.

3. Procédé de traitement amincissant par ionophorèse suivant l'une des revendications 1 ou 2, caractérisé en ce qu'une couche (9) de matière végétale est disposée autour de la ou des parties du corps (C) à traiter, entre la peau et la face interne de la ou des électrodes enveloppantes (2, 2a, 2b, 2c, 2d, 2e,...., 2n).

4. Procédé de traitement amincissant par ionophorèse selon la revendication 3, caractérisé en ce que la couche de matière végétale (3) est constituée par un tissu de coton, se présentant, par exemple, sous forme de bandelette apte à être enroulée autour de la partie du corps à traiter.

5. Dispositif pour l'application de traitements amincissants par ionophorèse ou de traitements amaigrissants par galvanothérapie, utilisant le passage d'un courant électrique fourni par un générateur (1), caractérisé en ce que ces électrodes (2, 2a, 2b, 2c, 2d, 2e,...., 2n) exécutées en caoutchouc conducteur, sont constituées par des enveloppes souples destinées à entourer des parties du corps humain et convenablement conformées pour épouser le plus étroitement possible les parties du corps (C) autour desquelles elles sont destinées à être placées.

6. Dispositif pour l'application de traitements amincissants par ionophorèse ou de traitements amaigrissants par galvanothérapie selon la revendication 5, caractérisé en ce que chaque électrode souple et enveloppante (2, 2a, 2b, 2c, 2d, 2e,...., 2n) constitue l'un des composants d'un ensemble morcelable apte à entourer complètement une ou des parties plus ou moins étendues du corps.

7. Dispositif suivant la revendication 6, caractérisé en ce chaque électrode souple et enveloppante (2, 2a, 2b, 2c, 2d, 2e,...., 2n) constitue l'un des composants d'une combinaison morcelable apte à envelopper complètement le corps, du cou aux chevilles.

8. Dispositif selon l'une quelconque des revendications 5 à 7, caractérisé en ce que au moins deux bords opposés de chaque électrode enveloppante (2, 2a, 2b, 2c, 2d, 2e,...., 2n) sont munis de moyens complémentaires d'assemblage rapide (3, 3a).

9. Dispositif selon l'une quelconque des revendications 5 à 8, caractérisé en ce que le ou les bords (5, 6) de chaque électrode enveloppante (2, 2a, 2b, 2c, 2d, 2e,...., 2n) destiné(s) à être placé(s) au contact ou à proximité du ou des bords d'une ou de deux électrodes contiguës, est ou sont pourvu(s) de moyens d'assemblage rapide (7, 7a) permettant de lier entre elles les différentes électrodes voisines de l'ensemble morcelable.

10. Dispositif suivant l'une quelconque des revendications 5 à 9, caractérisé en ce que chaque électrode enveloppante (2, 2a, 2b, 2c, 2d, 2e,...., 2n) est munie d'une pluralité de bornes de connexion (8) permettant de la relier électriquement à une ou plusieurs autres électrodes enveloppantes de l'ensemble morcelable.

**Claims**

1. A method of slimming treatment by ionophoresis, utilising a flow of electric current through the body, by means of flexible electrodes applied to the skin, characterised in that the electric current is transmitted through the body by means of enveloping electrodes (2, 2a, 2b, 2c, 2d, 2e,...., 2n) made of conductive rubber and arranged around the part or parts of the body to be treated, these enveloping electrodes being shaped so as to fit as closely as possible said part or parts of the body around which they have been placed.

2. A method of slimming treatment by ionophoresis according to claim 1, characterised in that an ionizable slimming substance or solution, intended to complete and/or improve the effectiveness of the treatment, is applied to the skin before the electric current is delivered.

3. A method of slimming treatment by ionophoresis according to either of claims 1 or 2, characterised in that a layer (9) of vegetable material is disposed around the part or parts of the body (C) to be treated, between the skin and the inner surface of the enveloping electrode or electrodes (2, 2a, 2b, 2c, 2d, 2e,...., 2n).

4. A method of slimming treatment by ionophoresis according to claim 3, characterised in that the layer (3) of vegetable material is composed of a cotton fabric which, for example, is in the form of a narrow strip or bandage to be wound around the part of the body to be treated.

5. A device for the application of slimming treatments by ionophoresis or weight-reducing treatments by galvanotherapy, utilising a flow of electric current supplied by a generator (1), characterised in that the electrodes (2, 2a, 2b, 2c, 2d, 2e,...., 2n), which are made of conductive rubber, comprise flexible coverings intended to encircle parts of the human body and suitably shaped to fit as closely as possible the parts of the body (C) around which they are to be placed.

6. A device for the application of slimming treatments by ionophoresis or weight-reducing treatments by galvanotherapy according to claim 5, characterised in that each flexible, enveloping electrode (2, 2a, 2b, 2c, 2d, 2e,...., 2n) forms one of the components of an assembly which can be separated into sections and which can completely surround one or more parts of the body separated from one another to a greater or lesser extent.

7. A device according to claim 6, characterised in that each flexible, enveloping electrode (2, 2a, 2b, 2c, 2d, 2e,...., 2n) forms one of the components of a combination which can be divided into sections and which can completely envelope the body from the neck to the ankles.

8. A device according to any one of claims 5 to 7, characterised in that at least two opposite edges of each enveloping electrode (2, 2a, 2b, 2c, 2d, 2e,...., 2n) are provided with additional rapid fastening means (3, 3a).

9. A device according to any one of claims 5 to 8, characterised in that the edge or edges (5, 6) of each enveloping electrode (2, 2a, 2b, 2c, 2d, 2e,...., 2n), intended to be brought into contact or into proximity with the edge or edges of one or two adjoining electrodes, is/are provided with rapid fastening means (7, 7a) making it possible to join together the various adjacent electrodes of the assembly which can be separated into sections.

10. A device according to any one of claims 5 to 9, characterised in that each enveloping electrode (2, 2a, 2b, 2c, 2d, 2e,...., 2n) is provided with a plurality of connecting terminals (8) enabling it to be connected electrically to one or more other enveloping electrodes of the assembly which can be separated into sections.

## Patentansprüche

1. Verfahren zur Schlankheitsbehandlung durch Ionophorese, bei der mittels weicher, auf die Haut aufgelegter Elektroden elektrische Ströme durch den Körper geleitet werden, dadurch gekennzeichnet, daß der elektrische Strom mittels einhüllender Elektroden (2, 2a, 2b, 2c, 2d, 2e, ..., 2n) geleitet wird, die aus leitfähigem Gummi bestehen und um den oder die zu behandelnden Körperteil(e) angeordnet sind, wobei diese einhüllenden Elektroden so gestaltet sind, daß sie sich an den oder die Körperteil(e), um den bzw. die sie angeordnet sind, so eng wie möglich anlegen.

2. Verfahren zur Schlankheitsbehandlung durch Ionophorese nach Anspruch 1, dadurch gekennzeichnet, daß eine ionisierbare, schlankmachende Substanz oder Lösung, die dazu bestimmt ist, die Wirksamkeit der Behandlung zu vervollständigen und/oder zu verbessern, vor dem Zuführen des elektrischen Stroms auf die Haut aufgebracht wird.

3. Verfahren zur Schlankheitsbehandlung durch Ionophorese nach einem der Ansprüche 1 oder 2, dadurch gekennzeichnet, daß eine Schicht (9) aus vegetabilischem Material um den oder die zu behandelnde(n) Körperteil(e) (C) zwischen der Haut und der Innenseite der umhüllenden Elektrode(n) (2, 2a, 2b, 2c, 2d, 2e, ..., 2n) angeordnet wird.

4. Verfahren zur Schlankheitsbehandlung durch Ionophorese nach Anspruch 3, dadurch gekennzeichnet, daß die Schicht aus vegetabilischem Material (3) von einem Baumwollgewebe gebildet ist, das sich beispielsweise in Bandform darstellt, die dazu geeignet ist, um den zu behandelnden Körperteil gewickelt zu werden.

5. Vorrichtung zur Verabreichung von Schlankheitsbehandlungen durch Ionophorese oder von Abmagerungsbehandlungen durch Galvanotherapie unter Durchleiten eines von einem Generator (1) gelieferten elektrischen Stroms, dadurch gekennzeichnet, daß diese Elektroden (2, 2a, 2b, 2c, 2d, 2e, ..., 2n), die aus leitfähigem Gummi bestehen, von weichen Hüllen gebildet sind, die dazu bestimmt sind, menschliche Körperteile zu umgeben, und vorzugsweise so gestaltet sind, daß sie sich an die Körperteile (C), um die sie angebracht werden sollen, so eng wie möglich anlegen.

6. Vorrichtung zur Verabreichung von Schlankheitsbehandlungen durch Ionophorese oder von Abmagerungsbehandlungen durch Galvanotherapie nach Anspruch 5, dadurch gekennzeichnet, daß jede weiche und einhüllende Elektrode (2, 2a, 2b, 2c, 2d, 2e, ..., 2n) eines der Bestandteile einer zerlegbaren Anordnung bildet, die dazu eingerichtet ist, einen oder mehrere mehr oder weniger ausgedehnte(n) Körperteil(e) vollständig zu umgeben.

7. Vorrichtung nach Anspruch 6, dadurch gekennzeichnet, daß jede weiche und einhüllende Elektrode (2, 2a, 2b, 2c, 2d, 2e, ..., 2n) eines der Bestandteile einer zerlegbaren Kombination bildet, die dazu geeignet ist, den Körper vom Hals bis zu den Knöcheln vollständig einzuhüllen.

8. Vorrichtung nach einem der Ansprüche 5 bis 7, dadurch gekennzeichnet, daß wenigstens 2 entgegengesetzte Ränder einer jeden einhüllenden Elektrode (2, 2a, 2b, 2c, 2d, 2e, ..., 2n) mit komplementären Schnellkupplungseinrichtungen (3, 3a) versehen sind.

9. Vorrichtung nach einem der Ansprüche 5 bis 8, dadurch gekennzeichnet, daß der Rand oder die Ränder (5, 6) einer jeden einhüllenden Elektrode (2, 2a, 2b, 2c, 2d, 2e, ..., 2n), der (die) dazu bestimmt ist (sind) in Berührung oder in die Nähe des Randes oder der Ränder von einer oder von zwei anschliesenden Elektroden gebracht zu werden, mit Schnellkupplungseinrichtungen (7, 7a) versehen ist oder sind, die es erlauben, die verschiedenen benachbarten Elektroden der zerlegbaren Anordnung miteinander zu verbinden.

10. Vorrichtung nach einem der Ansprüche 5 bis 9, dadurch gekennzeichnet, daß jede einhüllende Elektrode (2, 2a, 2b, 2c, 2d, 2e, ..., 2n) mit mehreren Verbindungsanschlüssen (8) versehen ist, die es erlauben, sie elektrisch mit einer oder mehreren anderen einhüllenden Elektroden der zerlegbaren Anordnung zu verbinden.

Fig.1

Fig.2

Fig.3

2a

2

2b

2d

Fig.4

2e

2n

Fig.5

1

2

2d

2n 2e

2a·2b